(19) Europäisches Patentamt
European Patent Office
Office européen des brevets

(11) Publication number: **0 431 169 A1**

# (12) EUROPEAN PATENT APPLICATION published in accordance with Art. 158(3) EPC

(21) Application number: **90901899.6**

(51) Int. Cl.[5]: **G01N 33/531**, G01N 33/543

(22) Date of filing: **19.01.90**

(86) International application number: **PCT/JP90/00058**

(87) International publication number: **WO 90/08320 (26.07.90 90/17)**

(30) Priority: **19.01.89 JP 8610/89**
**14.04.89 JP 93178/89**
**21.06.89 JP 156659/89**

(43) Date of publication of application: **12.06.91 Bulletin 91/24**

(84) Designated Contracting States: **AT BE CH DE ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **HOSODA, Kenji**
**3-3-12, Asahi-cho Kawagoe-shi**
**Saitama 350(JP)**
Inventor: **KUBOTA, Takaharu**
**3-18-4-211, Tamadaira Hino-shi**
**Tokyo 191(JP)**
Inventor: **HONDA, Hitomi**
**3-18-4-223, Tamadaira Hino-shi**
**Tokyo 191(JP)**
Inventor: **HINO, Shuichiro**
**1-28-4-308, Ozu-cho Iwakuni-shi**
**Yamaguchi 740(JP)**
Inventor: **HASEGAWA, Ryoichi**
**1-28-5-302, Ozu-cho Iwakuni-shi**
**Yamaguchi 740(JP)**
Inventor: **ITOU, Kazuhiko**
**3-1-3-2, Yamate-cho Iwakuni-shi**
**Yamaguchi 740(JP)**

(74) Representative: **Kraus, Walter, Dr. et al**
**Patentanwälte Kraus, Weisert & Partner**
**Thomas-Wimmer-Ring 15**
**W-8000 München 22(DE)**

## (54) IMMUNOASSAY METHOD, REAGENT AND KIT.

(57) This invention relates to an immunoassay method and reagents and kits for use therein, wherein a minute amount of a protein present in a specimen is assayed by an immunoreaction using a solid phase carrier prepared by immobilizing an antibody on an insoluble carrier and a labeled antibody prepared by combining an antibody with a labeled substance, characterized in that said immunoreaction is conducted in the presence of fowl sera. This method allows a minute amount of a protein present in a human specimen, especially a coagulated fibrinolytic protein, to be assayed with a high sensitivity.

EP 0 431 169 A1

# IMMUNOLOGICAL ASSAYING METHOD, REAGENT AND KIT THEREFOR

## Technological Field

This invention relates to an immunological assaying method of a trace amount of protein contained in an assay sample, and a reagent and a kit therefor, particularly to a method of immunologically assaying a trace amount of a target protein in a human assay sample in which relatively large amounts of proteins structurally similar to the target protein exist in addition to the target protein to be assayed, and to a reagent and a kit therefor and to a series of techniques relating to it.

## Background of Technology

An immunologically assaying method using an antibody is a widely used method because of the high specificity and high sensitivity. Since the discovery of a monoclonal antibody in 1975, the development of this method was expected more and more to be developed.

In order that this immunological assaying method should have high sensitivity, it is essential that a specific reaction of an antigen-antibody reaction alone should be high and other non-specific reactions should be as low as possible.

Particularly, where the amount of an antigen present in an assay sample is very small, or where substances similar to the antigen are present in relatively large amount, to inhibit a non-specific reaction as much as possible is necessary to increase the measurement sensitivity. To inhibit this non-specific reaction is an important techical point to complete a high sensitive immunoassay system, and for this reason, various attempts have been made from the past. A usually known means for it is to add an additive for inhibiting a non-specific reaction to the immunoassay system.

Some aspects of this means will be described.

(1) Japanese Laid-Open Patent Publication No. 182169/1982:

This Laid-Open Patent Publication describes that in an immunological reaction, a sample to be assayed is treated with a polyanion soluble in a reaction medium, such as dextran sulfate, heparin, polystyrenesulfonic acid, cellulose phthalate acetate, hyaluronic acid or chondroitin sulfate.

(2) Japanese Laid-Open Patent Publication No. 187862/1983:

This Laid-Open Patent Publication describes a method of increasing the measuring sensitivity by adding a synthetic surface-active agent such as nonionic surface-active agents, anionic surface-active agents or cationic-active agents to the immunological reaction system in the measurement of physiologically active substances by the immunoassay method.

(3) Japanese Laid-Open Patent Publication No. 36964/1985

This Publication describes a method of bio-chemical detecting method, which comprises treating a surface with at least one agent which limits a non-specific binding between a ligand or an anti-ligand and another substance, said agent being

(i) a surface-active agent which has one aromatic residue and having an HLB number of at least 16,
(ii) amphoteric ion surface-active agent or
(iii) a solution containing a salt of a polyvalent anion in a concentration of at least 100 mM.

(4) Japanese Laid-Open Patent No. 71861/1987

This Patent Publication describes a method of assaying an immunological reaction component, which is present an a solid phase, at a temperature of 15 to 40 °C, characterized in that a surface-active agent having an HLB value of greater than 20 is added. This Publication states that by adding the surface-active agent having the above HLB value, the immunological reaction is promoted, and without strengthening the dissociation of a reaction component, undesirable side reaction such as the non-specific adsorption of conjugate to a solid is inhibited.

(5) Japanese Patent Publication No. 25184/1984

This Patent Publication describes a method of removing non-specific inhibitory actions in an immunological assay, which is characterized in that in determining the trace amounts of components in a living body by immonological assay, by conjointly causing at least 0.1 % of hydrophobic protein and 0.2 M to 1.0 M of salts to inhibit the effects of interfering substances contained in a biological assay sample. As the hydrophobic substances, the Patent Publilcation illustrate collagen hydrolyzates, gelatin and lipoprotein lipase, as the hydrophobic proteins, and sodium chloride and phosphates as the salts.

(6) Japanese Laid-Open Patent Publilcation No. 65162/1986

This Publication describes a method of immunological assay using a mouse monoclonal antibody in which mouse serum, mouse ascites, rat serum, or rat ascites is caused to be present as a non-specific reaction absorber, and a non-specific reaction absorber comprising the above serum or ascites.

In the above Patent method, if a monoclonal antibody derived from mice (IgG or IgM) is used as a solid-phase and/or a labelled antibody, the presence of an anti-mouse immuloglobulin (for example anti-mouse IgG or IgM) in the assay sample would induce reaction with the monoclonal antibody on the solid phase and/or labelled antibody, and even an assay sample which is antigen-negative shows the results as if it were positive with the antigen. To absorb such a non-specific reaction with the anti-mouse immunoglobulin, the mouse serum, mouse ascites, rat serum or rat ascites is used. The above Patent Publication states that other additives such as goat serum, horse serum, bovine serum, porcine serum, rabbit serum, dog serum, chicken serum, physilogical saline, PSB or gelatin, have little action to absorb the above-mentioned non-specific reactions.

The above-mentioned methods of inhibiting non-specific adsorption reactions have their own defects, and are difficult to be said to be satisfactory methods where it is necesssary to measure traces of target substances accurately. Specifically, the methods described in (1) to (4) involves the use of various surface active agents which are non-proteinous substances. But since methods inhibit not only non-specific adsorption reactions but also the specific reactions originally intended, they are not suitable as methods for determining trace amounts of target proteins.

On the other hand, the method involving using the hydrophobic proteins together with salts as described in the Publication mentioned in (5) has not been found to have an effect of inhibiting a non-specific adsorption reaction in the immunoassay system intended by the present inventors.

The method involving using sera or ascites of mice and rats as described in the Patent Publication of (6) above have a problem in reproducibility and homogeneity especially when the ascites are used. Depending upon the source of procurement, reaction may be decreased, and this by itself is against the intended object of the immunological assay.

Generally, animal sera, particularly sera of mammals frequently are used to inhibit a non-specific adsorption reaction in an immunoassay system of a human assay sample.

However, to use the serum of a mammal for immunoassay of a human assay sample, the first thing to observe is that the animal serum should not contain a substance having cross reactivity with the target protein. Investigations of the present inventors have shown that in the case of determining a trace amount of a protein in an assay sample, such as a coagulation fibrinolysis system protein, since the mammal serum contains various substances which cross-react with them, an attempt to inhibit a non-specific absorption reaction gave rise to several problems. To explain these problems specifically, a thrombin-antithrombin III complex, an important protein typical of the coagulation fibrinolysis system protein is taken up as an example, and the problems in determining this complex will be described below in detail.

An antithrombin III (to be sometimes, referred to as "ATIII") is an important inhibitor of serine protease in the blood coagulation system, and inhibits not only thrombin but also activated XII, XI, X and IX factors or kalicrein and plasmin. The reaction of ATIII and serine protease proceeds at a mole ratio of 1:1, and the arginine residue of ATIII binds to the serine residue of the active center of serine protease through an ester linkage to form a complex whereby the activity of the serine protease is suppressed. One example of such a complex is thrombin-antithrombin III complex ( to be referred to as "TAT"). The presence and increase of TAT in the blood are considered to be a indicator of formation of thrombin by the starting and activation of the blood coagulation mechanism.

Generally, the blood of a healthy person contains about 110,000 to 230,000 ng/ml of prothrombin and about 200,000 to 500,000 ng/ml of ATIII. On the other hand, the concentration of TAT is as small as about 1 to 3 ng/ml. However, it is said that in the blood of a patient with brain infraction, the concentration of TAT is about 10 ng/ml, and the TAT level in the blood of a DIC patient, it is about 30 ng/ml (see, for example, "The Japanese Journal of Clinical Pathology, vol. 36, NO. 6, pages 623 to 626 (1988)). Thus, in thrombus formation and its cource, the TAT concentration of human blood is apparently higher than in healthy persons.

Accordingly, by measuring the amount of TAT in the blood, a part of the condition of the blood coagulation system can be known, and by this, it is expected to elucidate the diseased condition of the patient from the aspect of the blood coagulation, predict in the early stage the development of the diseased condition to, for example, thrombus formation or DIC (disseminated intravascular coagulopathy) and to be able to perform an adequate therapy.

Heretofore, as an attempt to measure TAT, a method using assaying an anti-TAT neoantigen antibody has been investigated. Specifically, Herbert K. Lau et al. attempted to measure TAT by the inhibition assay using anti-TAT neoantigen antibody and $^{125}$I-labelled TAT. [see The Journal of Biological Chemistry, Vol.255, 5885, Issue of Jan. 25 (1980)].

Pelzer et al. studied a TAT assay system by a sandwich system using an anti-thrombin antibody as a solid-phase antibody and anti-ATIII antibody as an enzyme labelled antibody (Thrombosis & Haemostasis, July 14 (1985).

However, these conventional mathods do not always meet the present-day therapeutic needs. For example, in the aforesaid method of Lau et al., the selectivity of the anti-TAT neoantigen antibody is a problem. This antibody has cross-reactivity with free thrombin, ATIII or prothrombin, and there is a possibility of the measured values drastically varying. The reason for this is that generally, the concentration of TAT in a human assay sample is only about $1/10^5$ of that of free ATIII or prothrombin. Furthermore, the method of Pelzer et al. has insufficient sensitivity. For this reason, it is difficult to dilute plasma to sufficiently low concentrations. This causes the defect that an assay system of plasma is prone to undergo plasma interference.

It was therefore desired to provide an assay system for specifically determine TAT with high sensitivity using a small amount of an assay sample.

Thus, when the present inventors added bovine serum to the above known TAT assay system, the bovine serum showed no effect of increasing sensitivity even when it is added to the TAT assay system. This is probably because the bovine serum contains a high concentration components having a high degree of cross-reactivity with human serum components, such as bovine ATIII and bovine TAT.

## MEANS FOR SOLVING THE PROBLEM

Accordingly, the present inventors continued to study additives for immunoassay which do not have cross-reactivity with a human assay component and be able to inhibit the non-specific adsorption reaction effectively in order to determine a trace amount of protein from a human assay sample containing trace amounts of proteins such as TAT, particularly containing large amounts of other proteins having a similar structure to the trace amount of protein.

As a result, the present inventors found that if, in an immunological assay method,the serum of fowl is present in the reaction solution of an immunological reaction, the desired specific reaction is not inhibited and non-specific adsorption reactions are sufficiently suppressed and therefore, the trace amount of protein in the assay sample can be determined with high sensitivity.

Thus, according to this invention, there is provided an immunological assay method of determining a trace amount of protein in an assay sample by an immunological reaction using a solid-phase antibody resulting from immobilizing an antibody to an insoluble carrier and a labelled antibody resulting from binding a labelling substance to an antibody, said method being characterized in that the immunological reaction is carried out in the presence of the serum of fowl, and a reagent and a kit therefor.

By this invention, a trace amount of a protein in an assay sample, particularly the trace amount of the target protein can be immunologically determined with high sensitivity from a human assay sample, particularly one containing a trace amount of protein and large amounts of other proteins having a similar structure to the trace amount of the target protein.

The present invention will be described further in detail hereinbelow.

In the immunological assay system of this invention, various proteins can be the target proteins in trace amounts which are to be determined. The present invention is particularly suitable for the determination of a trace amount of a target protein in a human assay sample containing large amounts of other proteins having a similar structure to the target trace protein as stated above.

The term "other proteins having a similar structure to the target trace protein" denotes proteins which have a region having partially the same amino sequences as the target trace protein and having a strong affinity for the solid-phase antibody or the labelled antibody in the immunological assay system.

Examples of such target proteins include coagulation fibrinolysis system proteins such as $\alpha_2$plasmin inhibitor ($\alpha_2$PI), $\alpha_2$-plasmin inhibitor-plasmin complex, protein C, protein S, tissue plasminogen activator-plasmin activator inhibitor complex, and thrombin-antithrombin III complex (TAT); hormones such as human chorionic gonadotropin (HCG), luteinizing hormones (LH), and follicle-stimulating hormone (FSH); tumor markers such as alpha-fetoprotein (AFP) and carcino-embryonic antigen (CEA); lung surfactant-apoprotein; and chondrocalcin etc. Among these target trace proteins, the coagularion fibrinolysis system proteins, especially the thrombin-antithrombin III complex (TAT), can be measured with very high sensitivity by the immonological assay method of this invention. Accordingly, the present invention is applied advantageously to the measurement of these proteins.

The sera of the fowl used in the immunological assay may be sera obtained from fowl such as chicken, geese, turkeys, quails, and doves. Of these the sera of chikens, geese or turkeys are preferred.

In the present invention, the serum of the fowl may be caused to be present in the immunological reaction system for immunologically assaying the target protein in the human assay sample. To make a detailed explanation on this point, a typical method of the immunological measuring method is generally called the sandwich method, and is largely divided into the one step method and two step method. The present invention can be applied to any of the one step and two step methods in the sandwich method. Specifically, the one step sandwich method is a method whereby a human assay sample containing the target protein, a solid phase antibody immobilized to an insoluble carrier and a labelled antibody are reacted immunologically in the same reaction system to form a complex of the solid-phase antibody, the target protein and the labelled antibody, and the amount of the labelled substance in the complex is measured to determine the amount of the target protein. On the other hand, the sandwich method by the two step method is the method whereby a human assay sample containing the target protein is first reacted with a solid-phase antibody immobilized to an insoluble carrier immunologically to form a complex of the solid-phase antibody and target protein (first immunoreaction), and then added a labelled antibody to form a complex of the solid phase antibody-target protein labelled antibody (second immunoreaction,) and the amount of the labelling substance in the complex is measure to determine the amount of the target protein.

According to this invention, the serum of the fowl is caused to be present in an immunological reaction in the one step method of the sandwich method, and in the first immunological reaction and/or in the second immunological reaction in the two step immunological reaction. Specifically, the serum of the fowl is added as such, or added as a mixture with an aqueous solution or a buffer. Alternatively, it may be added beforehand to a human sample or a labelled antibody solution.

In the present invention, by causing the serum of the fowl to be present in the aforesaid immunological reaction system, a non-specific adsorption reaction can be effectively suppressed. Furthermore, the intended specific reaction is carried out without a cross reaction. Consequently, the trace amount of the target protein can be assayed with high sensitivity.

On the other hand, the present inventors found that if a bovine serum, a porcine serum, a sheep serum, a rat serum, a horse serum, and a rabbit serum are used for the assaying of a trace amount of protein in a human assay sample, even if the non-specific adsorption reaction can be lowered, a cross reaction is liable to take place, they are inadequate as additives for immunological assay with high sensitivity. For example, if the material to be assayed is a coagulation fibrinolysis system protein, in particular, when these animal sera are used for the assay system of thrombin-antithrombin III complex (TAT), a cross reaction takes place.

Accordingly, the trace amounts of prorteins to be assayed in this invention are not particularly limited. Examples include protreins which are liable to induce cross reaction with , for eample, anti-sera, for example coagulation fibrinolysis system proteins, particularly the aforesaid TAT. Now, by taking up a method of assaying TAT as an example, the method of immunological assay, the immonological assay reagent, and the kit therefor will be described. These are given merely for the sake of illustration, and the present invention should not be limited to the determination of TAT.

(A) Method of immulologically assaying human-thrombin-ATIII complex (TAT)

An antibody to human thrombin or an equivalent fragment thereof (first antibody) is first immobilized to a suitable insoluble carrier (for example, a plastic container) (to be referred to as a "solid-phase antibody"). Then, to avoid a non-specific binding between the insoluble carrier and a reagent or an assay sample used for the assay, a suitable substance (for example, bovine serum albumin) is coated on the surface of the insoluble carrier.

The resulting solid-phase antibody is contacted and reacted with the human assay sample at a fixed temperature for a certain period of time.

By adding the serum of the fowl to the reaction solution during this immunological reaction (the first immunological reaction), only the non-specific adsorption reaction can be lowered without inhibiting the specific reaction. The concentration of the serum of the fowl actually used is 1 to 100 v/v% , preferably 5 to 50 V/v%. During this time, the solid-phase antibody is bound to the human thrombin-ATIII complex (TAT).

Then, after washing with a suitable washing solution, an antibody to human ATIII or an equivalent fragment thereof (second antibody) which is labelled with a suitable labelling substance i.e., a solution containing a labelled antibody such as an aqueous solution is contacted and reacted with a human-thrombin-ATIII complex (TAT) bounded to a solid-phase antibody at a fixed temperature for a certain period of time. By performing this imunological reaction (second immunological reaction) in the presence of the serum of fowl, the specific reaction can be maintained as such while effectively suppressing the non-specific adsorption of the labelled antibody. The concentration in which the serum of the fowl is used at this time is finally 1 to 100 v/v%, desirably 3 to 30 v/v% in the immunological reaction solution. After the labelled antibody is reacted with the TAT bound to the solid-phase antibody, the reaction product is washed with a washing solution. Then, the amount of the labelling substance labelled on the second antibody present on the insoluble carrier is measured. Thus, the amount of the human thrombin-ATIII complex (TAT) in the human assay sample can be calculated. In the foregoing, the sandwich method in which the immunological reactions are carried out by a first immunological reaction and a second immunological reactrion have been described. The assay method of this invention is not to be limited to the two-step method, and can be equally applicable to the aforesaid one-step method.

(B) Make-up of the assay reagent and kit

As a reagent for the immunological assay of human thrombin-ATIII complex (TAT) there may be (a-1) the aforesaid solid phase antibody and (a-2), the aforesaid labelled antibody, one or both of which may be combined with the serum of fowl or a solution containing it.

Furthermore, the kit for immunological assay of human thrombin-ATIII complex (TAT) comprises the assay reagents (a-1), (a-2) and auxiliary agents for using these assay reagents efficiently with simplicity and ease, such as (b) dissolving agents for dissolving solid reagents or liquid assay samples (c) washing agents used for washing antigens and antibodies bound non-specifically to insoluble carriers, and (d) where antibodies labelled with enzymes are used, a substrate for measuring enzyme activity and a reaction stopper therefor, and (e) other substances normally used for immonological assaying. Examples of the insoluble carriers used in the immunological assaying method and reagents and kits of human-thrombin-ATIII complex (TAT) shown as one example of the invention include high polymers such as polystyrene, polyethylene, polypropylene, polyesters, polyacrylonitrile, fluorine resins, crosslinked dextran and polysaccharides, in combination with paper, glass, metals, agarose, and combination of these.

The insolble carriers may be in various forms such as trays, spheres, fibrous shapes, rods, discs, containers, cells and test tubes.

As a labelling substance of the labelled antibodies, it is advantageous to use enzymes, fluorescent substance, light emitting substances and radioactive substances. The enzymes include, for example, peroxidase, alkaline phosphatase, and beta-D-galactosidase. The fluorescent substances include, for example, fluorescein isothiocyanate, and phycobilli protein. The luminicent substances may include isolucinol and lucigenin. The radioactive substances may be, for example, $^{125}I$, $^{131}I$, $^{14}C$, and $^{3}H$. These illustrations are not limited, and may be those which can be used in immunological assay.

When the labelling substances are enzymes, substrates may be used to measure its activity, and as required, coloring agent may be used.

When peroxidase is used as an enzyme, $H_2O_2$ is used as the substrate and 2,2'-azinodi-]-[3-ethylbenzo-thiazoline sulfate ]ammonium salt (ABTS), 5-aminos-alicylic acid, o-phenylenediamine, 4-aminoantipyrine, and 3,3',5,5'-tetramethylbenzidine may be used. When alkaline phosphatase is used as an enzyme, o-nitrophenyl phosphate is used as the substrate. When beta-D-galactosidase is used as an enzyme, fluoresein- di(beta-D-galactopyranoside) and 4-methylumbelliferyl-beta-D-galactopyranoside may be used as a substrate.

Further, in the immunological assaying kits, the dissolving agets (b) may be any of those which are usually employed for immunological assay. For example, those which include a phosphate buffer, a Tris-HCl buffer, or an acetate buffer and having a pH of 6.0 to 8.0 may be shown as preferred examples. As the (c) washing agents, those which are generally used for immunological asay may be used as such. Examples include physiological saline, phosphate buffer, Tris-HCl buffer and mixtures thereof. To these washing agents, there may be added nonionic surface-active agents such as Trion X100, Tween 20 and Brig 35, and ionic surface-active agents such as sodium dodecylsulfate.

The above immunological assaying methods, assay reagents, and kit therefor have been described as the serum of fowl is applied to the measurement of thrombin-antithrombin III complex (TAT) in a human assay sample. The use of the serum of fowl in this inveniton is not at all limited to the measurement of TAT, and it may be achieved the same purpose and effect for the measurement of other traces of proteins.

Thus, according to this invention, the following method of immunological assay, the following reagets and kits are provided.

Immonological assaying method

A method of assaying a trace amount of protein in an assay sample by an immunological reaction using a solid-phase antibody resulting from immobilizing an antibody to an insoluble carrier and a labelled antibody resulting from binding a labelling substance to an antibody.

Immunological assay reagent

A reagent for immunologically assaying a trace amount of protein in an assay sample by immunological reaction using a solid phase antibody (a-1) obtained by immobilizing an antibody to an insoluble carrier and a labelled antibody (a-2) obtained by binding a labelling substance to an antibody, wherein the serum of fowl or its solution is combined with the solid-phase antibody (a-1) or the labelled antibody (a-2), or both.

Immunological assay kit

An immunological assay kit for immunologically assaying a trace amount of protein in an assay sample comprising

(a-1) a solid phase antibody immobilized an antibody to an insoluble carrier
(a-2) a labelled antibody composed of an antibody and a labelling substance bound thereto,
(b) a dissolvng agent,
(c) a washing agent, and
(d) if the labelling substance is an enzyme, a substrate for measuring the enzyme activity, and a reaction stopper, characterized in that at least one of the solid phase antibody (a-1), the labelled antibody (a-2) and the dissolving agent (b) is further combined with the serum of fowl or a solution containing it.

In the aforesaid immunological assaying method and the assay system including the reagent and kit, the antibody in the solid-phase antibody and the labelled antibody may be any of polyclonal antibodies or monoclonal antibodies. Furthermore, these antibodies may be not only complete antibodies but also fragments thereof having the same recognition sites. Preferably, the fragments thereof may be a Fab' fragment or a $F(ab')_2$ fragment thereof.

In the immunoassay system described above, the insoluble carrier, the labelling substance, the dissolving agent, the washing agent and the serum of fowl may be the same as described with respect to the immunological assay system of the aforesaid thrombin-antithrombin III complex (TAT).

The investigations of the present inventors have led to the discovery that in the immunological assaying system for a trace amount of protein, especially a coagulation fibrinolysis system protein, in a human assay sample, the conjoint use of the serum of fowl makes it possible to assay a target protein with high sensitivity, but that by properly combining it with the following technical embodiments, better and more stable sensitivity can be achieved.

(1) Use of a carrier having excellent surface smoothness as the insoluble carrier.
(2) Use of a solution containing an amphoteric surface-active agent or a surface-active agent having an HLB of at least 16 as the washing agent.
(3) Addition of a specific protein in an immunological reaction.

By combining at least one of the embodiments (1) to (3) with the immunological assay system of this invention, non-specific raactions are further inhiibited, and the trace amount of protein in the human assay sample can be assayed with very high sensitivity and stability. In the following these technical embodiments (1) to (3) will be described.

(1) Use of an insoluble carrier having excellent surface smoothness as the insoluble carrier:

It has been found that when in the assay of a trace amount of protein intended in this invention, the use of a carrier having a mirror surface with smootness as the insoluble carrier makes it possible to suppress non-specific adsorption reactions of proteins in the assay sample or labelled antibodies to the carrier and the assay sensitivity increases and stability also increases as compared with a carrier having a rough surface.

In the past, to enhance the assay sensitivity in an immunological assay system, an insoluble carrier having a rough surface obtained by grinding its surface to increase its surface area has been used. Certainly, when an assay sample contains a relatively large amount of target protein, or it does not contain another protein having a similar structure to the target protein, the use of an insoluble carrier having a rough surface is rational and effective. However when an assay sample contains only a very small amount of a target protein such as TAT and another protein having a similar structure is present conjoitly in the assay sample, a non-specific adsorption is inhibited as the smoothness of the surface increases, and the assay sensitivity increases.

Thus, advantageously, the insoluble carrier has a smooth mirror surface having a centerline average roughness (Ra) of its surface of not more than 1.5 micrometers.

The centerline average roughness (Ra) means the value of Ra given by the following formula in microns when from the roughness curve the measuring length portion in the direction of the centerline is pulled out and the centerline of the pulled out portion is designated as X-axis and the direction of vertical times is designated as Y-axis, and the roughness curve is expressed by y = f(x).

$$Ra = \frac{1}{\ell}\int_0^{\ell} /f(x)/dx$$

This centerline average roughness (Ra) is described in JIS B 0601-1982 (Japan), ANSI B 46.1 - 1978 (USA) and R 468-1966 (ISO).

In examples of the present invention, the surface roughness of the insoluble carrier is measured by using a surface roughness tester, SURFCOM® (made by Tokyo Seimitsu Co., Ltd.)

The material and shape of the insoluble carrier having the above smooth surface are not particularly limited, and those explained above will be shown. An especially preferred example is a polystyrene bead.

(2) Use of a solution containing an amphoteric surface-active agent or a surface-active agent having an HLB of at least 16:

Generally, in immunological assay, an antigen or a labelled antibody non-specifically remaining or being adsorbed to a solid phase or a test tube after the immunological reaction is removed by an operation of washing. As such a washing agent, water, physiological saline, or a phosphate buffer is usually used.

To perform washing effectively in order to lower a non-specific adsorption and increase the assay sensitivity, it was proposed to increase the number of washings or increase the amount of the washing agent, or the washing method or conditions are specially determined minutely. However, even by these means, a satisfactory washing effect is not always achieved. Especially, for the measurement of a very trace amount of an antigen, or for assaying a specific protein in an assay sample in which a protein of a similar structure is coexisting, the washing means or the washing solution greatly affects the assaying sensitivity.

Thus, the investigations of the present inventors have led to the discovery that by using a washing agent containing not more than 1 w/w% and having a pH of not more than 6 of an amphoteric surface-active agent and/or a surface-active agent having an HLB of at least 16, the washing effect is increased, and non-specific adsorptions can be decreased by a fewer washing operation without requiring special conditions or operations.

The present inventors studied the case of using a normally used washing agent having a pH of 7. Consequently, the so-called background values (the absorbance when the concentration of the antigen is zero) could be lowered to some degree. But it was difficult to obtain an elevating effect of the measureing sensitivity when the concentration of the antigen was in the very low concentration range of ng/ml or pg/ml. It was not sufficient for the purpuse of reducing non-specific adsorptions and assaying the target protein with high sensitivity. To increase the assay sensitivity, it is important to decrease non-specifc adsorptions as much as possible and lower the background value as much as possible. When in this case, it was attempted to increase the concentration of the surface-active agent, but it often suppressed specific reactions and it was impossible to increase the sensitivity. The present inventors noted the pH of the washing agent, and adjusted it to not more than 6 instead of the conventonal pH 7. Then surprisingly, by adding a small amount of the surface active agent, the background value could be lowered without inhibiting specific reactions, and the sensitivity could be increased.

The surface-active agents used as the washing agents are amphoteric surface-active agents or surface-active agents having an HLB of at least 16.

Examples of the amphoteric surface-active agents include carboxylc acid salt type (betaine type), sulfonic acid salt type (sulfobetaine type) and phosphoric acid salt type. The sulfobetaine type sometimes poses a problem of carcinogenity, and its use is not always commercially preferred. The amphorteric surface-active agents of the betaine type are preferred. They are of the betaine type expressed by the general formula $R_1R_2R_3NR_4$ wherein $R_1$ represents an alkyl group having 8 to 22 carbon atoms, $R_2$ and $R_3$ both represent alkyl groups having 1 to 6 carbon atoms, and $R_4$ represents an alkyl group having 1 to 6 carbon atoms having a $COO^{\ominus}$ substituent. Especially preferred are compounds of the above general formula wherein $R_2$ and $R_3$ are methyl groups, and $R_4$ is $CH_2COO^{\ominus}$, and $R_1$ is a lauryl group. Preferred surface active agents of this type are sold under the tradename UNHITOL 24B (tradename of Kao Co., Ltd.).

Examples of surface-active agents include, for example, surface-active agents such as polyoxyalkylene alkylaryl ethers, polyoxyalkylene alkyl ether, polyoxyalkylene polyol fatty acid esters and polyoxyethylene polyoxypropylene polyols having an HLB value of at least 16, specifically including Triton X-305 (HLB value = 17.3), Triton X-405 (HLB value = 17.9), Emulgen 950 (HLB value = 18.2), Emulgen 985(HLB value = 18.9), Tween 20 (HLB value = 16.7), Pulronic F68 (HLB = 29), and Tetronic 707 (HLB value>20).

Anionic surface-active agents may have an HLB value of at least 16 such as carboxylic acid salts, sulfonic acid salts, sulfuric acid ester salts and phosphoric acid ester salts including, for example, sodium n-dodecylsulfate (HLB values = 40 and sodium tetradecylsulfate (HLB value = 39).

These surface-active agents may be used alone, or at least two types of surface-active agents may be used together, as in the case of using amphoteric surface active agents and anionic surface-active agents concurrently.

The concentration of the surface-active agent in the washing agent may be not more than 1 w/w%, prefer ably 0.001 w/w% to not more than 0.05 w/w%. Even if the amount of the surface-active agent is not more than 0.05 w/w %, it has excellent washing effect and bubbling of washing is reduced at the time of washing. Accordingly, the use of these surface-active agent can obviates the problem that the bubbling is vigorous and it is troublesome to eliminate the bubbles or its elimination becomes insufficient, and conversely, the washing is insufficient. Hence, the use of these surface-active agents is favorable.

The washing agent contains the above surface-active agent, and its pH is adusted to 6 or below, preferably to 4 or more.

Examples of the solvent used for the washing agent may be those materials which do not adversely affect the assaying, for example, water, physiological saline, and buffers such as a phosphate-citrate buffer.

(3) Addition of a specific protein in an immunological reaction:

It has been found that if in the immunological assay system of the invention, a protein having a molecular weight of 16,000 to 50,000 and an isoelectric point of 1.0 to 5.0 or a mixture containing them is caused to be present in the immunological reaction soltuion and the final concentration of the protein in these immunological reaction solution is adjusted to 0.02 to 0.9 % by weigt, non-specific adsorptions are suppressed, and therefore, the background becomes low and high sensitivity tends to be easy to obtain. Such a protein or a mixture containingit may be included in the above amount in the immunological reaction solution in the immunologial assay reagent constituting a part of the reagent and the kit used in the immunological assaying method of the present invention. Examples of such protein include casein, pepsin, ovoglycoprotein, and orosomucoid. (Such a mixture may contain 10 to 60 % by weight, preferably 20 to 50 % by weight, of the above protein, 30 to 80 % by weight, preferably 40 to 60 % by weight, of a sugar (such as lactose), fats (for example 2 to 2 % by weight) ash (for example 5 to 12 % by weight) and water content (for example 2 to 8 % by weight). A typical example of such a mixture is skim milk. The skim milk contains casein as a protein. As compared with the use of casein alone, skim milk has good dispersibility in the immunologcal reaction solution, has a high effect of decreasing a non-specific adsorption and has good preservability at a temperature of 4 °C (difficulty of forming a precipitate). The skim milk may be derived from any material if it is defatted milk. One of the most typical skim milk is commercially available from Difco Co., Ltd.

As stated above, according to the present invention, the coagulation fibrinolysis system protein in a human assay sample may be assayed stably with high sensitivity.

The investigations of the present inventors have led to the discovery that in the case of an assay system of human thrombin-antithrombin III complex (TAT), one of the very important protein of the coagulation fibrinolysis system proteins, a trace amount of TAT can be assayed with still high sensitivity by selecting an anti-human-thrombin anibody or an equivalent fragment immobilized to an insoluble carrier as a solid-phase antibody and a human antithrombin III antibody or an equivalent fragment thereto bound to a labelling substance as a labelled antibody.

Thus, according to the present invention, there are provided a method of assaying TAT in a human assay sample, and a reagent and kit therefor, which are described in (I) to (III).

(I) A method of assaying a human thrombin-antithrombin III complex (TAT) in a human assay sample by an immunological reaction using a solid phase antibody resulting from by immobilizing an anti-human-thrombin antibody or equivalent fragment thereof to an insoble carrier and an a labelled antibody resulting from binding a labelling substance to antihuman-antithrombin III antibody or an equivalent fragment thereof bound to a labelling substance, wherein said immunological reaction is carried out in the presence of the serum of fowl.

(II) A reagent for immunologically assaying a human thrombin-antithrombin III complex (TAT) in a human assay sample comprising (a-1) a solid phase antibody an anti-human thrombin antibody or an equivalent fragment thereof to an insoluble carrier and (a-2) a labelled antibody resulting from binding a labelling substance to an anti-human anti- thrombin III antibody or an equivalent fragment thereof, characteirzed in that the serum of fowl or a liquid containing it is combined with one or both of the solid phase antibody (a-1) or the labelled antibody (a-2).

(III) An immunological assaying kit for immunologically assaying a human thrombin-antithrombin III complex (TAT) in a human assay sample comprising a combination of

(a-1) a solid phase antibody resulting from immobilizing an anti-human thrombin antibody or an equuivalent fragment thereof to an insoluble carrier,

(a-2) a labelled antibody resulting from binding labelling substance to an anti-human antithrombin III antibody or an equivalent fragment thereof,

(b) a dissolving agent,

(c) a washing agent, and

(d) if the labelling substance is an enzyme, a substrate for measuring an enzyme activity and a reaction stopper, characterized in that the serum of fowl or a liquid containing it is combined with at least one of the solid phase antibody (a-1), the labelled antibody (a-2), and the dissolving agent (c).

In the Immunological assay system for TAT described in (I) to (III), examples of the anti-human thrombin antibody used as a solid phase antibody include anti-human thrombin polyclonal antibodies, anti-human thrombin monoclonal antibodies and fragments of these antibodies having equivalent functions to these antibodies, such as $F(ab')_2$, Fab' or Facb. Of these, the anti-human thrombin polyclonal antibodies are preferred. Such polyclonal antibodies can be obtained by immunizing an animal with human thrombin as an antigen, and purifying the resulting anti-serum using a thrombin-immobilized column by a conventional known method (see, for example, Pelzer et al., Thrombosis and Haemostatis 59 (1), 101 - 106, 1988). Of these, preferably used are a rabbit anti-human thrombin polyclonal antibody, and a goat anti-human thrombin polyclonal antibody.

As a solid phase for immobilizing such an anti-human thrombin antibody, it is preferable to use insoluble carriers with a surface having a centerline average roughness (Ra) of not more than 1.5 micrometers. By using such an insoluble carrier having a smooth surface, non-specific adsorptions by free human antithrombin III in the human assay sample are tremendously reduced, and TAT can be assayed with higher sensitivity.

On the other hand, in the aforesaid TAT assay system, examples of the anti-human anti-thrombin III antibodies as labelled antibodies may include anti-human antithrombin III polylonal antibody, anti-human antithrombin III monoclonal antibody and fragments of these antibodies having equivalent functions, such as $F(ab')_2$, Fab' and Facb. Of these, anti-human antithrombin III polyclonal antibody is preferred from the standpoint of sensitivity. Examples of such anti-human antithrombin III polyclonal antibody may be obtained by immunizing a rabbit or a goat with human antithrombin III by a known method. Especially, when Fab' obtained by decomposing such anti-human antithrombin III polyclonal antibody with pepsin in accordance with the method of Nisonoff et al.(see A. Nissonoff et al., Arch, Biochem. Biophys. 89 230, 1960) and subjecting the resulting $F(ab')_2$ to a reducing reaction is used, an assay system with higher sensitivity can be obtained preferably.

Binding of a labelling substance to such an anti-human antithrombin III antibody or its fragment may be carried out by a known method such as the glutalaldehyde method and the maleimide method. Particularly, it is preferred to bind the labelling substance via the sulfur atom of an antibody. In this case, the labelling substance may be increased in reactivity to the sulfur atom by maleimidizing it.

As stated above, in the present invention, a trace amount of protein can be assayed with high sensitivity by carrying out the immunologial reaction in the presence of the serum of fowl. According to another finding of the present inventors, it was found that the serum of fowl is very effective for the preparation of a labelled antibody.

Specifically, it has been found that generally a labelled antibody is used as a reagent by lyophilization. A dried antibody containing the serum of fowl obtained by lyophilizing a labelled antibody in the presence of the serum of fowl can be prepared stably as compared with the preparation in the absence of the serum of fowl and a labelled antibody of stable quality can be obtained.

Thus, according to this invention, there can be provided a stabilized labelled antibody or its fragment containing the serum of fowl. The above-mentioned stabilizing effect is especially superior when the antibody is an antibody to a coagulation fibrinolysis system protein or its fragment such as Fab' or $F(ab')_2$.

Brief Description of the Drawings

Figure 1 attached show the effects given to TAT assay, which were examined when the sera of various animals were added to immunological reactions;

Figure 2 shows the concentration of the serum of chicken added in an immunological reaction and the effects given to TAT assay; and

Figure 3 shows the results of the effect of adding the serum of chicken in the lyophililzed antibody.

(f) Examples

The present invention will be described in detail by Examples.

In the Examples, polyclonal antibodies obtained by known methods were used as an antihuman thrombin antibody and an anti-human ATIII antibody.

REFERENTIAL EXAMPLE 1

Composition of a thrombin-antithrombin III complex assay system:

(1) Labelling of an anti-human ATIII antibody Fab' with horseradish peroxidase (HRP)

To a PBS solution of 2 mg/ml of an anti-human ATIII antibody, 100 microliters of 1M acetate buffer (pH 4.2) and a solution of 40 micrograms of pepsin in 20 tion was reacted at 37 °C for 16 hours. After the termicrolitersof the same buffer were added, and the solumination of the reaction, the reaction product was separated by using Sephadex G-25 column (2 cm in diameter x 45 cm) which had been equilibrated with 0.1M phosphate buffer (pH 6.0) containing 5 mM EDTA to collect $F(ab')_2$. Then, $F(ab')_2$ was reduced with mercaptoethylamine, and gel-filtered using a Toso G3000 SW column (HPLC) to purify Fab'.

On the other hand, the maleimidized HRP synthesized using M-maleimide benzoyl-N-hydroxy succinimide ester (MBS) was isolated. Finally, Fab' was mixed with maleimidized HRP and the mixture was concentrated using a Filtron (ultrafiltration unit). The reaction was carried out at 4 °C overnight and the HRP-labelled anti-human ATIII antibody Fab' were isolated and purified by using Toso-G3000 SW.

(2) Assay of human thrombin-ATIII complex (TAT) by a sandwitch enzyme immunoassay.

One polystyrene bead (Ra = 0.8 micrometer) to which an anti-human thrombin antibody was immobilized and 350 microliters of a 0.5% BSA-PBS solution (pH 7.4) 0 ng/ml to 10 ng/ml of purified human TAT were added to each test tube, and incubated at 37 °C for 20 minutes. The solution in the test tube was aspirated and removed, and washed with PBS. The HRP-labelled antibody prepared in section (1) was added to each test tube and incubated at 37 °C for 20 minutes. Then, the solution in the test tube was aspirated and removed, and the test tube was washed with PBS. Then, 0.4 of 0.1M phosphate citrate buffer (pH 4.0) containing 0.02 % of 3,3',5,5'-tetra-methyl benzidine hydrochloride and 0.005 % of $H_2O_2$ were put into each test tube and incubated at 37 °C for 20 minutes, and then as a reaction stopper 1 ml of a 1N sulfuric acid was added to stop the enzyme reaction.

Then, the absorbance of this solution at a wavelength of 450 nm was examined by using a spectrophotometer, and a calibration curve was prepared.

EXAMPLE 1

Measurement of substnces having cross reactivity in the sera of various animals:

Each animal serum described in Figure 1 was added to systems not containing TAT so that the final serum concentration became 10 %. In accordance with the assay method of Referential Example 1, an immunological reaction was carried out. The absorbance was measured, and from the measured absorbances, the human TAT concentrations (ng/ml) of the animal serum were determined, and are indicated in Figure 1. The results showed that the sera of the fowl including chiken, geese, turkeys, quails, and doves (ng/ml) contained no substances having cross-reactivity with human TAT. Furthermore, it has been found that sera of bovine, swine, goats and horses contained substances which have cross reactivity with human TAT.

EXAMPLE 2

Effect of adding the serum of chicken in assaying of TAT (1):

By adding the serum of chicken in an amount of 0, 10, 20, and 30 v/v% in a first immunological reaction, and in accordacnce with the assaying method of Referential Example 1 a immunological reaction was carried out, using TAT solution in an amount of 0, 10, and 20 ng/ml, and an assay sample taken from a healthy person after diluting it to four times. The results are shown in Figure 2. As shown in Figure 2, when a human assay sample was used, a non-specific adsorpion was decreased by adding the serum of chicken and the specific reaction was maintained.

EXAMPLE 3

Effect of adding the serum of chicken in TAT assay (2):

In the TAT assay described in Example 1, the effect of adding the serum of chicken in a second immunological reaction was investigated. Specifically, in Example 2, TAT = 2.5 ng/ml was used, and a first immunological reaction was carried out by adding 10 v/v % of the serum of a chicken, and in a second immunological reaction, three types of enzyme-labelled antibodies were used (i.e., (a) an enzyme-labelled antibody not subjected to lyophilization, (b) a lyophilized enzyme-labelled antibody, and (c) an enzyme-labelled antibody lyophilized in the presence of 10 v/v% of the serum of a chicken). The results are shown in Table 3. The CV value at 0 ng/ml is taken as 10 %. The TAT conoentration at a point where the value of [OD value + 3SD] at 0 point crosses a straight line connecting a TAT concentration of 0 ng/ml to a TAT concentration of 2.5 ng/ml is defined as the measuring sensitivity. The measuring sensitivities are also indicated in Figure 3. As shown in Figure 3, it is seen that by adding the serum of chicken (c), a non-specific adsorption of an enzyme-labelled antibody is effectively inhibited by lyophilization, and a specific immunological rection was maintained.

EXAMPLE 4

[Studies of the sensitivities of an enzyme-labelled antibodies]

(1) Preparation of enzyme-labelled antibodies [IgG, $F(ab')_2$, Fab']

(a) Labelling of anti-human ATIII antibody IgG with HRP

Fifty microliters of a dimethyl formamide solution of N-(m-maleimide benzoic acid)-N-succinimide ester (MBS) in a concentration of 10 mg/ml was added to a 1.0 mg/ml PBS solution of anti-human ATIII antibody IgG, and the mixture was reacted at a temperature of 25 °C for 30 minutes. Then by using a column filled with Sephadex G-25, the reaction mixture was subjected to gel filtrationusing a 0.1M phosphate buffer (pH 6.0) to separate the maleimidized antibody and the unreacted MBS.

On the other hand, an ethanol solution in a concentration of 10 mg/ml of N-succinimidyl-3-(2-pyridylthio)propionate (SPDP) was added to a 1.0 mg/ml PBS solution of HRP, and the mixture was reacted at 25 °C for 30 miniutes. Then by using a column filled with sephadex G-25, the reaction mixture was purified by gel filtration with a 0.01 M acetate buffer (pH 4.5) to collect a fraction containing pyridyl disulfide HRP, and concentrated to about 10 times in a collodion pack under ice cooling. Then, 1 ml of 0.1M acetate buffer (pH 4.5) containing 0.85 % NaCl and 0.1M dithiothreitol was added, and the solution was stirred at 25 °C for 20 minutes to reduce the pyridyl disulfide group introduces into HRP molecule. Then, the reaction mixture was gel-filtered in a column of Sephadex G-25 column to obtain a fraction containing thiolated HRP. Then, the above maleimidized antibody was mixed with the thiolated HRP, and the mixture was concentrated to a protein concentration of 4 mg/ml using a collodion pack under ice cooling, followed by allowing the mixture to stand at 4 °C for one day. Then, the mixture was gel-filtered using a column filled with ultrogel AcA44 (LKB Co., Ltd.) to obtain a HRP-labelled anti-human AT III antibody IgG fraction.

(b) Labelling of an anti-human ATIII antibody $F(ab')_2$ with HRP

100 microliters of an acetate buffer (pH 3.7) and a solution of 40 micrograms of pepsin in 20 microliters of the same buffer were added to a PBS solution of 2.0 mg/ml of an anti-human ATIII antibody, and the solution was reacted at 37 °C for 3 hours. After the reaction, the reaction product was separated by using a Sephadex G-25 column (2 cm in diameter x 45 cm) equilibrated with PBS to collect $F(ab')_2$. The preparationof HRP labelled anti-human ATIII antibody $F(ab')_2$ was performed in accordance with Example 4, (1) (a).

(c) Labelling of anti-human ATIII antibody Fab' with HRP

100 microliters of 1M acetate buffer (pH 4.2) and a solution of 40 micrograms of pepsin in 20 micro liters of the same buffer were reacted with a PBS solution of 2 mg/ml of anti-human ATIII antibody at 37 ° for 16 hours. After the reaction, the reaction mixture was separated by using a Sephadex G-25 column (2 cm in diameter x 45 cm) equilibrated with 0.1 M phosphate buffer (pH 6.0) containing 5 mM of EDTA to collect $F(ab')_2$. Then, $F(ab')_2$ was reduced with mercaptoethylamine, and Fab' was purified by gel filtration HPLC on Toso G3000 SW column.

On the other hand, by using MBS, maleimidized HRP was isolated. Finally, while Fab' was mixed with maleimidized HRP, concentration was performed by using a Filtron (ultrafiltration unit), and the reaction was performed. By performing the reaction overnight at 4 °C, HRP-labelled anti-human ATIII antibody Fab' was isolated and purified by Toso G 3000 SW HPLC.

(2) Assay of human thrombin ATIII complex (TAT) by a sandwich enzyme immunoassay method:-

One polystyrene bead (Ra=0.8 micrometer) to which human thrombin antibody was immobilized and 400 microliters of a 0.5% BSA/PBS solution (pH 7.4) containing the purified human TAT in the range of 0 ng/ml to 10 ng/ml and containing 30 % of chicken serum were added to a test tube, and incubated at 37 °C for 20 minutes. Then, the solution in the test tube was aspirated and removed. The test tube was washed with PBS, and then each of the three HRP-labelled antibodies (prepared in (a), (b) and (c) of section (1) was added to the each test tube, and incubated at 37 °C for 20 minutes. Then, the solution in the test tube was aspirated and removed, and the test tube was washed with PBS. Further 0.1 M phosphate citrate buffer (pH 4.0) containing 0.0225 % of 3,3',5,5'-tetramethylbenzidine hydrochloride and 0.009 % of $H_2O_2$ added to each test tube in an an amount of 0.4 ml, and incubated at 37 °C for 20 minutes. Then, as a reaction stopper, 1 ml of a 1N sulfuric acid was added to stop the enzyme reaction.

Then, by using a spectrophotometer, the absorbance at a wavelength 450 nm of the solution was measured. By combination with various labelled antibodies, the N/S ratios (%) where N is an OD value at a TAT concentration of 0 ng/ml, and S is an OD value at a TAT concentration of 10 ng/ml. The results are shown in Table 1.

Table 1

| Fractions of HRP-labelled antibody | $OD_{450}$ (N) (TAT=0 ng/ml) | $OD_{450}$ (S) (TAT=10 ng/ml) | N/S (%) Sensitivity (*) |
|---|---|---|---|
| IgG [Example 4, (1)(a)] | 0.062 | 1.033 | 6.0 (192 pg/ml) |
| $F(ab')_2$ [Example 4, (1)(b)] | 0.023 | 0.713 | 3.2 (100 pg/ml) |
| Fab' [Example 4, (1)(c)] | 0.018 | 0.685 | 2.6 (81 pg/ml) |

* sensitivty (the measured values in accordance with the definition of Example 3.

It is seen from Table 1 that when Fab' and F(ab')$_2$ were used as the labelled antibodies, the non-specific adsorptions were reduced, and the sentivitities were particularly superior.

EXAMPLE 5

[Studies of sensitivities depending upon the roughness of the surface of a solid phase antibody]

By using polystyrene beads for EIA having various centerline average roughness shown in Table 3 below, anti-human thrombin antibodies were immobilized to obtain solid phase antibodies. On the other hand, PBS solutions containing 25% of TAT-deficient plasma and 20% of chicken serum at the concentration of TAT of 0 ng/ml and 20 ng/ml were preapred.

By using these solid phase antibodies and the prepared PBS solution, and further by using anti-human ATIII antibody as an enzyme-labelled antibody, TAT was assayed in accordance with Example 4 (2). The measured sensitivities of the polystyrene beads with various centerline average roughnesses (Ra) of the surface were as shown in Table 2.

Table 2

| Polystyrene bead | Ra (micrometers) | Sensitivity |
|---|---|---|
| A | 5.2 | 222 pg/ml |
| B | 3.9 | 204 pg/ml |
| C | 1.3 | 138 pg/ml |
| D | 0.8 | 90 pg/ml |
| E | 0.6 | 73 pg/ml |

It is seen from the results shown in Table 2 that the use of insoluble carriers having a smooth surface with a Ra of not more than 1.5 micrometers permits assay of TAT with even higher sensitivity.

EXAMPLE 6

In an asasy system of thrombin antithrombin III complex (TAT) by the sandwich method, the effect of a surface active agent used as a washing agent was examined.

(1) Labelling of anti-human antithrombin III (ATIII) antibody Fab' with HRP:

To 20 ml of a PBS solution containing anti-antithrombin III antibody of 7 mg/ml (pH 7.2) 2 ml of a 1M acetate buffer (pH 4.2) was added. Then, 540 microliters of the same buffer containing 2.7 mg of pepsin was added, and reacted at 37 °C for 5 hours. After the end of the reaction, the reaction mixture was separated by a Sephadex G-25 column (2 cm in diameter x 45cm) equilibrated with 0.1 M phosphate buffer (pH 6.0) containing 5 mM of EDTA and F(ab')$_2$ was collected. Then, F(ab')$_2$ was reduced with mercapoethylamine, and Fab' was purified by gel filtration HPCL by a TOSO G3000 SW column.

On the other hand, HRP was maleimidized using SMCC (succinimide 4-(N-maleimidomethyl)-cyclohexane-1-carboxylate), and maleimidized HRP was isolated. Finally, Fab' was mixed with maleimidized HRP reaction mixture was concentrated using a Filtron (ultrafiltration unit) and reacted at 4 °C overnight. HRP-labelled anti-human ATIII antibody Fab' was isolated and purified on OSC G 3000 SW. The resulting Fab', with the addition of chicken serum in a concentration of 10%, was lyophilized.

(2) Assay of human thrombin ATIII complex (TAT) by the sandwich immunoassay method

One polystyrene bead (Ra = 0.8 micrometer) having anti-human thrombin antibody immobilized thereto, and 400 microliters of a 0.1% skim milk-0.5% BSA-30% chicken serum-PBS solution having a purified human TAT in a concentration of 0 ng/ml and 20 ng/ml, were added to a test tube, and inbcubated at 37 °C for 20 minutes. Then, the solution in the test tube was sucked and removed. The test tube was washed twice with 3 ml of physiological saline containing 0.0025 % of each of the surface-active agents indicated in Table 2 with its pH adjusted to 5.5 and 7.0. After the concentration of HRP-labelled antibody Fab' obtained in (1) was adjusted to 1 microgram/ml with 0.1% skim milk-0.5% BSA-PBS, 400 μl of this solution added to a test tube, and incubated at 37 °C for 20 minutes. Then, the solution in the test tube was aspirated and removed, and washed with 3 ml of the same washing solution as in the washing after the first reaction. After washing, 400 microliters of 0.1 M phosphate citrate buffer containing 0.0225 % of 3,3',5,5'-tetra-methylbenzidine hydrochloride and 0.009 % of $H_2O_2$ was added to each test tube, and incubated at 37 °C for 20 minute. Then, as a reaction stopper, 1 ml of a 1N sulfuric acid was added to stop the enzyme reaction.

Then, the absorbance of this solution at 450 nm was measured by using a spectrophotometer. As in Example 4, the N/S ratio was determined when various washing agents were used.

The results are shown in Table 3. As is clearly seen from Table 3, as compared with the case where the pH of the washing agent containing the surface active agent was 5.5, the absororbance at 0 ng/ml and the N/S ratio were higher when the pH was 7.0. Accordingly pH values of not more than 6.0 were advantageous.

Table 3

| Washing agent | | | TAT 0 ng/ml Absorbance (N) | TAT 20 ng/ml Absorbance (S) | N/S ratio (%) |
|---|---|---|---|---|---|
| Surface-active agent | Concentration(%) | pH | | | |
| Lauryl dimethyl aminoacetic acid betaine | 0.0025 | 5.5 | 0.020 | 1.372 | 1.5 |
| Stearyl dimethyl aminoacetic acid betaine | " | " | 0.022 | 1.386 | 1.6 |
| Polyoxyethylene sorbitan monolaurate [Tween 20] (HLB=16.7) | " | " | 0.018 | 1.222 | 1.5 |
| Polyoxyethylene octylphenyl ether [P8P] (HLB=14.0) | " | " | 0.030 | 1.304 | 2.3 |
| Lauryl dimethyl aminoacetic acid betaine | " | 7.0 | 0.033 | 1.432 | 2.3 |
| Stearyl dimethyl aminoacetic acid betaine | " | " | 0.038 | 1.380 | 2.8 |
| Polyoxyethylene sorbitan monolaurate | " | " | 0.056 | 1.643 | 3.4 |

EXAMPLE 7

As washing agents, lauryldimethylaminoacetic acid betaine in saline (pH 5.5) having each of the concentrations shown in Table 6 were used, and by operating in the same way as in Example 6 the effects of the concentrations used were examined. The results are shown in Table 4.

It is seen from Table 4 the N/S ratio was small over a range of 1.0 to 0.001 %, and the assay sensitivity increased in a low concentration range.

When it was 2 %, bubbling was vigorous, and the absorbance at a TAT of 20 ng/ml was markedly lowered.

Table 4

| Concentration of lauryl dimethyl acetic acid betaine (%) | TAT 0 ng/ml Absorbance (N) | TAT 20 ng/ml Absorbance (S) | N/S ratio (%) |
|---|---|---|---|
| 0 | 0.036 | 1.340 | 2.7 |
| 0.0001 | 0.034 | 1.343 | 2.5 |
| 0.001 | 0.022 | 1.400 | 1.6 |
| 0.0025 | 0.019 | 1.352 | 1.4 |
| 0.005 | 0.020 | 1.306 | 1.5 |
| 0.01 | 0.019 | 1.300 | 1.5 |
| 0.05 | 0.018 | 1.296 | 1.4 |
| 0.1 | 0.019 | 1.330 | 1.4 |
| 0.5 | 0.017 | 1.224 | 1.4 |
| 1.0 | 0.012 | 1.020 | 1.1 |

EXAMPLE 8

Effect of adding the serum of chicken in the assay of human plasmin-$\alpha_2$ plasmin inhibitor complex (PIC):

(1) Preparation of a solid phase antibody

IgG component was separated and purified from anti-serum to human plasminogen obtained by immunizing human plasminogen with rabbit. The resulting anti-plasminogen antibody was adjusted with PBS to a protein concentration of 20 micrograms/ml. Polystyrene beads (Ra = 3.9 micrometers) were added, and by a known method, plasminogen antibody-immobilized polystyrene beads were obtained.

(2) Preparation of a labelled antibody

In the same way as in Example 4, (a), HRP-labelled anti-$\alpha_2$-plasmin inhibitor antibody was obtained by using anti-$\alpha_2$.plasmin inhibitor monoclonal antibody obtained by the method of Example 3 described in Japanese Laid-Open Patent Publilcation No. 222426/1985. The resulting labelled antibody was diluted with PBS (pH 7.2) containing 0.5 % BSA to a labelled antibody concentration of 0.4 micrograms/ml to prepare a labelled antibody solution (A). On the other hand, in the same way, the labelled antibody was diluted with PBS containing 0.5 % BSA to and 10 % chicken serum, to prepare a labelled antibody solution (B).

(3) Assay of PIC by the sandwich enzyme immunoassay method

200 microliters of 0.5% BSA-PBS solution (pH 7.2) containing 0 ng/ml and 50 ng/ml of purified human plasmain-$\alpha_2$-plasmin inhibitor (PIC) was taken into each of test tubes, and to the solution 200 microliters of the labelled antibody (A) or the labelled antibody (B) obtained in (2). Then one polystyrene bead to which was immobilized the anti-plasminogen antibody obtained in (1) was added to each test tube, and incubated at 37 °C for 60 minutes. Then, the solution in the test tube was aspirated and removed, and the test tube was washed with physiological saline. Then, 0.1M phosphate citrate buffer (pH 4.5) containing 1.5 % ABTS (3,3'-azino-di-1-ethylbenzothiazolinesulfonic acid (6)]) and 1 mM $H_2O_2$ were added to each test tube in an amount of 400 microliters, and incubated at 37 °C for 30 minutes. Then, 1 ml of 0.25 M oxalic acid was added to stop the enzyme reaction. Then, by using a spectrophotometer the absorbance of this solution at 420 nm was measured. The results are shown in Table 5.

Table 5

| Solution of HRP-labelled antibody | $OD_{420}$ (N) (PIC 0 ng/ml) | $OD_{420}$ (S) (PIC 50ng/ml) | N/S (%) * (Sensitivity) |
|---|---|---|---|
| 0.5% BSA-PBS solution | 0.234 | 1.255 | 18.6 (688 pg/ml) |
| 10% chicken serum 0.5% BSA-PBS solution | 0.053 | 1.391 | 3.8 (119 pg/ml) |

* Sensitivity (the measured values in accordance with the definition of Example 3)

It is seen from Table 5 that when the serum of chicken was added to the immunological reaction system, the non-specific adsorption was lowered, and the assay sensitivity was excellent.

## Claims

1. A method of immunological assay, which comprises assaying a trace of protein in an assay sample by immunological reaction using a solid phase antibody resulting from immobilizing an antibody to an insoluble carrier and a labelled antibody resulting from binding a labelling substance to an antibody, wherein the immunological reaction is carried out in the presence of a serum of fowl.

2. The method of claim 1 wherein the serum of fowl is contained in an amount of 1 to 100 v/v% in the immunologidal reaction system.

3. The method of claim 1 wherein the serum of fowl is contained in an amount of 5 to 50 v/v% in the immunological reaction system.

4. The method of claim 1 wherein the trace amount of protein in the assay sample is a coagulation fibrinolysis system protein.

5. The method of claim 1 wherein the trace amount of protein in the assay sample is human thrombin antithrombin III complex.

6. The method of claim 1 wherein the assay sample is a human serum or plasma.

7. The method of claim 1 wherein the serum of fowl is the serum of chicken, goose or turkey.

8. The method of claim 1 wherein the insoluble carrier has such surface smoothness that its surface has a centerline average roughness (Ra) of not more than 1.5 micrometers.

9. The method of claim 8 wherein the insoluble carrier is polystyrene or copolystyrene.

10. The method of claim 1 wherein a washing agent containing an amphoteric surface-active agent and/or a surface-active agnet having an HLB value of at least 16 in a concentration of not more than 1 w/w % and having a pH of not more than 6 is used.

11. An immunological assay reagent for assaying a trace of protein immunologically in an assay sample, said reagent comprising (1-a) a solid phase antibody resulting frrom immobilizing an antibody to an insoluble carrier and (a-2) a labelled antibody resulting from binding a labelling substance to an antibody wherein a solid phase antibody or a labelled antibody or both are combined with a serum of fowl or a liquid containing it.

12. The assay reagent of claim 11 wherein the trace of protein in the assay sample is a coagulation fibrinolysis system protein.

13. The assay reagent wherein the trace amount of protein in the assay sample is human thrombin antithrombin III complex.

14. The assay reagent of claim 11 wherein the insoluble carrier has such surface smoothness that its surface has a centerline average roughness (Ra) of not more than 1.5 micrometers.

15. The assay reagent of Claim 11 wherein the serum of fowl is the serum of chicken, goose or turkey.

16. An immunological assay kit for immunologically assaying a trace amount of protein in an assay sample, comprising

(a-1) a solid phase antibody resulting from immobilizing an antibody to an insoluble carrier,
(a-2) a labelled antibody resulting from binding a labelling substance to an antibody,
(b) a dissolving agent,
(c) washing agent, and
(d) where the labelling substance is an enzyme, a substrate for measuring the enzyme activity and a reaction stopper, wherein at least one of the (a-1) the solid phase antibody (a-2) the labelled antibody and the (b) dissolving agent is further combined with a serum of fowl or a liquid containing it.

17. The assay kit of claim 16 wherein the trace amount of protein in the assay sample is a coagulation fibrinolysis system protein.

18. The assay kit of claim 16 wherein the trace amount of protein in the assay sample is human thrombin antithrombin III complex.

19. The assay kit of claim 16 wherein the insoluble carrier has such surface smoothness that its surface has a centerline average roughness (Ra) of not more than 1.5 micrometers.

20. The assay kit of claim 16 wherein the serum of fowl is the serum of chicken, goose or turkey.

21. The assay kit of claim 16 wherein the washing agent contains an amphoteric surface-active agent and/or a surface-active agent having an HLB value of at least 16 in a concentration of not more than 1 w/w % and having a pH of not more than 6, or a concentrated solution thereof.

22. A method of assaying a human thrombin antithrombin III complex in a human assay sample by immunological reaction by using a solid phase antibody resulting from immobilizing an anti-human thrombin antibody or an equivalent fragment thereof immobilized to an insoluble carrier and an anti-humanantithrombin III antibody or an equivalent fragment to which a labelling substance is bound, characteirzed in that said immunological reaction is carried out in the presence of a serum of fowl.

23. The method of clalm 22 wherein the serum of the fowl is the serum of chicken, goose or turkey.

24. The method of claim 22 wherein the insoluble carrier has such surface smoothness that its surface has a centerline average roughness (Ra) of not more than 1.5 micrometers.

**25.** The method of claim 22 wherein in said assay, a washing agent containing an amphoteric surface-active agent and/or a surface-active agent having an HLB value of at least 16 in a concentration of not more than 1 w/w % and having a pH of not more than 6 is used.

**26.** The method of claim 22 wherein the anti-human thrombin antibody or an equivalent fragment thereof is an human thrombin polyclonal antibody or its Fab', $F(ab')_2$ or Facb fragment.

**27.** The method of claim 22 wherein the anti-human antithrombin III antibody or its equivalent fragment is anti-human antithromhin III polyclonal antibody or its Fab' or $F(ab')_2$ fragment.

**28.** The method of claim 22 wherein the human assay sample is a human serum or plasma.

**29.** The method of claim 22 wherein the labelling substance is an enzyme.

**30.** The immunological assay reagent for immunologically assaying a human-thrombin antithrombin III complex in a human assay sample comprising (a-1) a solid phase antibody resulting from immobilizing an anti-human thrombin antibody or its equivalent fragment to an insoluble carrier and (a-2) a labelled antibody resulting from binding a labelling substance to an anti-human antithrombin III antibody or an equivalent fragment wherein the solid phase antiboey (a-1) or the labelled antibody (a-2) or both are combined with a serum of fowl or a liquid containing it.

**31.** A kit for immunologically assaying a human-thrombin antithrombin III complex in a human assay sample comprising

(a-1) a solid phase antibody resulting from immobilizing anti-human thrombin antibody or an equivalent fragment immobilized to an insoluble carrier,

(a-2) a labelled antibody resulting from binding a labelling substance to an anti-human antithrombin III antibody or its equivalent fragment,

(b) a dissolving agent,

(c) a washing agent, and

(d) wherein the labelling substance is an enzyme, a substrate for measuring the enzyme activity and a reaction stopper wherein at least one of the solid phase antibody (a-1), the labelled antibody (a-2) and the dissolving agent (c) is combined with a serum of fowl or a liquid containing it.

**32.** A stabilized labelled antibody or its fragment containing teh serum of fowl.

**33.** The labelled antibody or its fragment according to claim 32 wherein the antibody or its fragment is an antibody to a human coagulation fibrinolysis system protein or its Fab' or $F(ab')_2$ fragment thereof.

**34.** A method of assaying a trace amount of protein in an assay sample by immunological reaction, wherein the immunological reaction is carried out in a presence of the serum of fowl.

FIG. 1

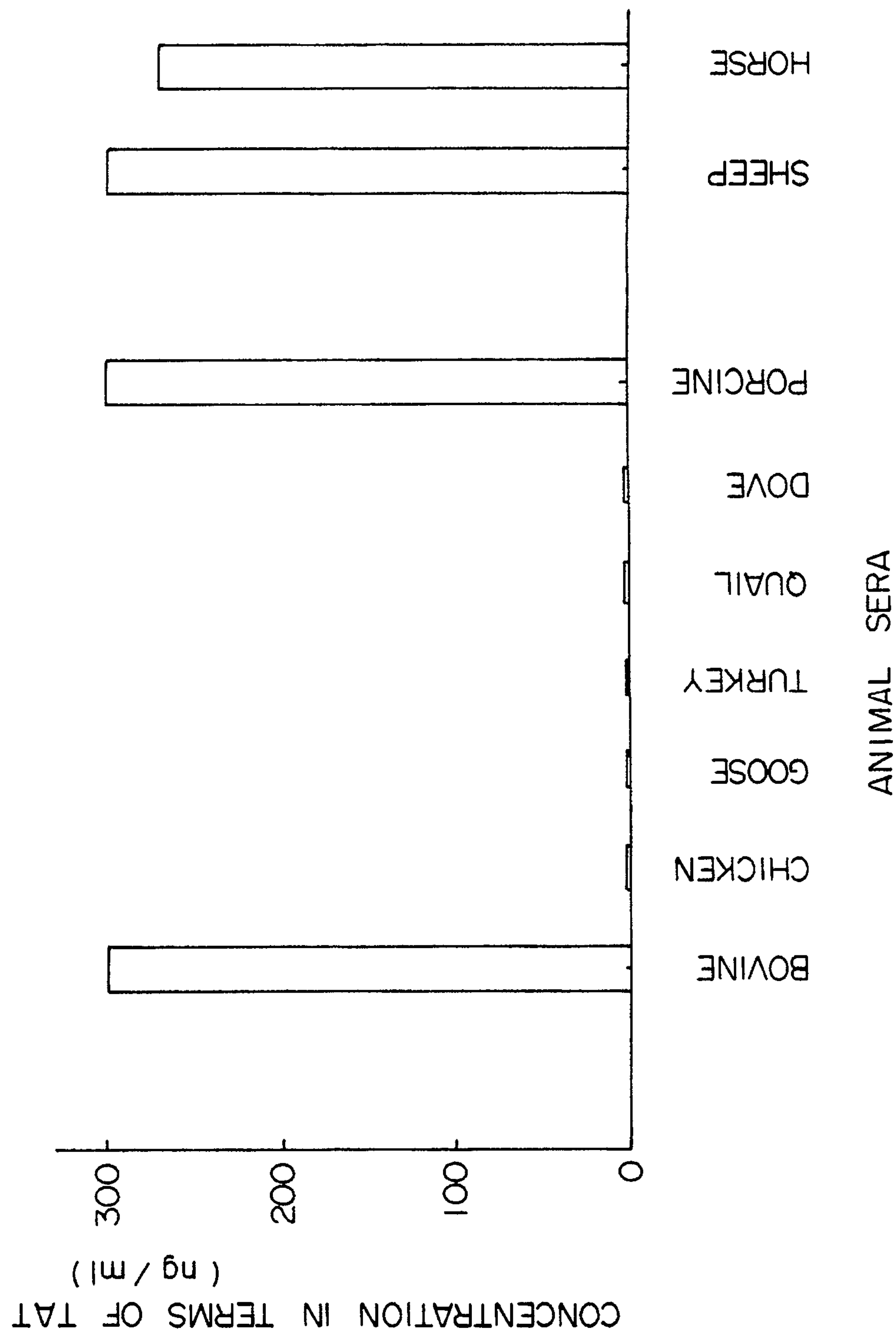
HORSE
SHEEP
PORCINE
DOVE
QUAIL
TURKEY
GOOSE
CHICKEN
BOVINE
ANIMAL SERA
0
100
200
300
CONCENTRATION IN TERMS OF TAT
( ng / ml )

FIG. 2

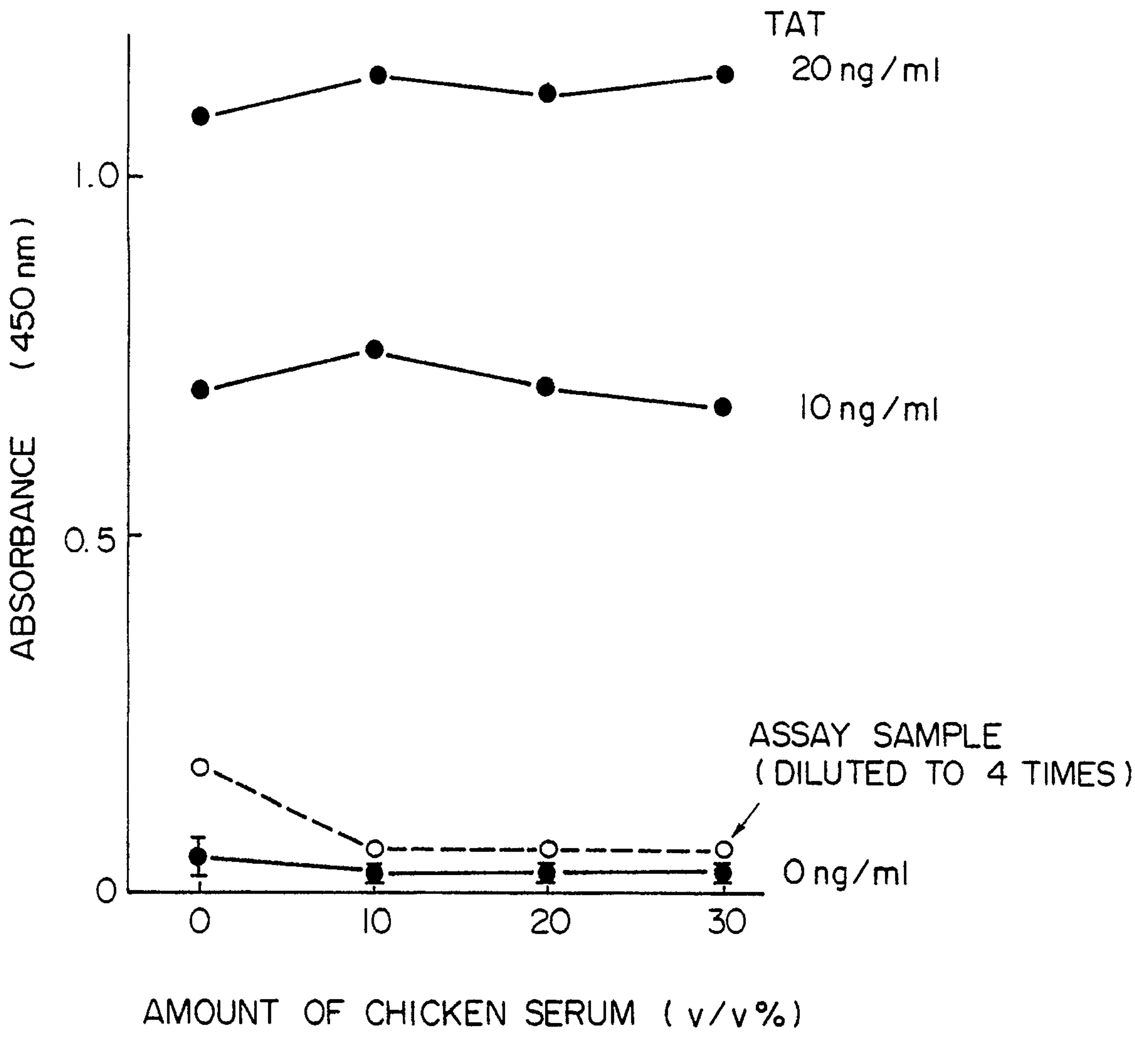
TAT
20 ng/ml
10 ng/ml
ASSAY SAMPLE
(DILUTED TO 4 TIMES)
0 ng/ml
1.0
0.5
0
0
10
20
30
ABSORBANCE (450 nm)
AMOUNT OF CHICKEN SERUM (v/v%)

FIG. 3

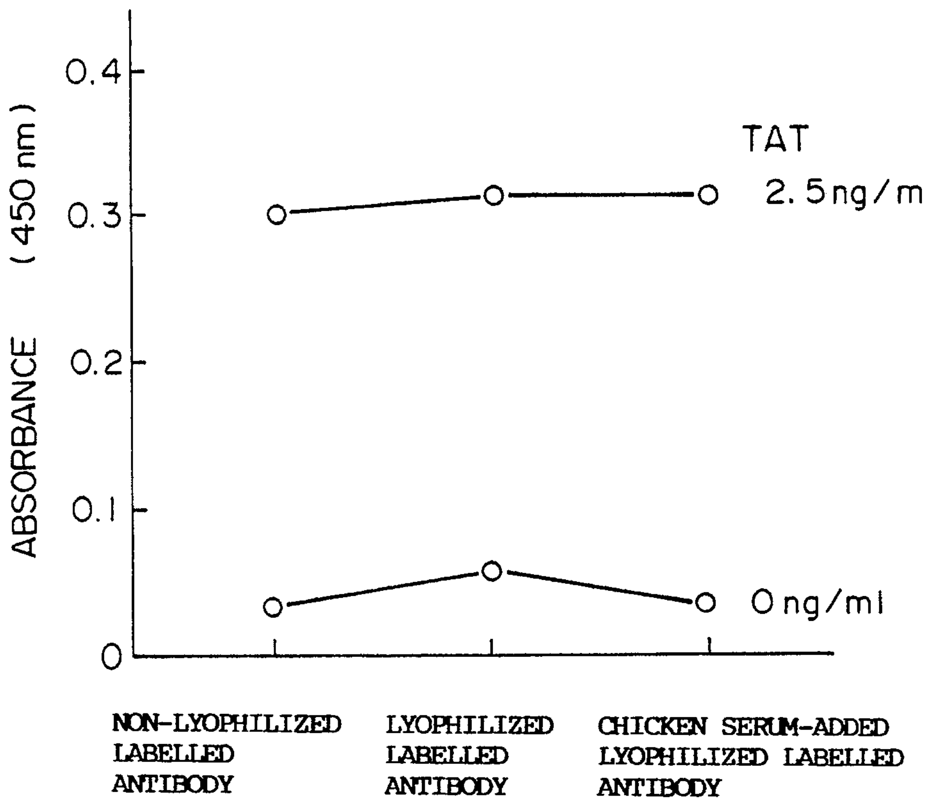


| | (a) | (b) | (c) | |
|---|---|---|---|---|
| ASSAY SENSITIVITY | 0.088 | 0.175 | 0.10 | TAT ng/ml |

# INTERNATIONAL SEARCH REPORT

International Application No PCT/JP90/00058

**I. CLASSIFICATION OF SUBJECT MATTER** (if several classification symbols apply, indicate all) [6]

According to International Patent Classification (IPC) or to both National Classification and IPC

Int. $Cl^5$ G01N33/531, G01N33/543

**II. FIELDS SEARCHED**

Minimum Documentation Searched [7]

| Classification System | Classification Symbols |
|---|---|
| IPC | G01N33/531, G01N33/543 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 62-138187 (Yatoron Kabushiki Kaisha), 20 June 1987 (20. 06. 87), (Family: none) | 1 - 34 |
| A | JP, A, 62-65693 (Wako Pure Chemical Industries, Ltd.), 24 March 1987 (24. 03. 87), (Family: none) | 1 - 34 |
| A | JP, A, 60-200169 (The Green Cross Corp.), 9 October 1985 (09. 10. 85), (Family: none) | 1 - 34 |
| A | JP, A, 55-152458 (Amano Pharmaceutical Co., Ltd.), 27 November 1980 (27. 11. 80), (Family: none) | 22 - 33 |
| X, Y | JP, A, 59-102161 (Zaidan Hojin Kagaku & Kessei Ryoho Kenkyusho), 13 June 1984 (13. 06. 84), (Family: none) | 1 - 34 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 28, 1990 (28. 03. 90) | April 9, 1990 (09. 04. 90) |
| **International Searching Authority** | **Signature of Authorized Officer** |
| Japanese Patent Office | |